# EUROPÄISCHE PATENTSCHRIFT

(11) **EP 1 820 796 B1**
(45) Veröffentlichungstag und Bekanntmachung des Hinweises auf die Patenterteilung: **06.01.2010**
(21) Anmeldenummer: 07000222.5
(22) Anmeldetag: 08.01.2007
(51) Int. Cl.: C07C 267/00, C07D 229/00

(54) **Verfahren zur Herstellung flüssiger, lagerstabiler Cardbodiimid, und/oder Uretonimingruppen aufweisender organischer Isocyanate**
Process for the preparation of fluid and storage stable organic isocyanates containing carbodiimide and/or uretonimino groups
Procédé pour la préparation d'isocyanates organiques liquides, stables au stockage, contenant des groupes carbodiimido et/ou urétonimino

(30) Priorität: 17.01.2006 DE 102006002158
(43) Veröffentlichungstag der Anmeldung: 22.08.2007
(73) Patentinhaber: Bayer MaterialScience AG, 51368 Leverkusen (DE)
(72) Erfinder: Wershofen, Stefan, Dr., 41065 Mönchengladbach (DE); Steinwegs, Marcus, 47829 Krefeld (DE); Schmidt, Manfred, Dr., 41540 Dormagen (DE)

(56) Entgegenhaltungen:
- EP-A- 1 607 425
- EP-A- 1 616 858
- EP-A2- 0 515 933
- FR-A1- 2 322 129
- FR-A1- 2 341 605

## Beschreibung

Die Erfindung betrifft ein Verfahren zur Herstellung flüssiger, lagerstabiler Carbodiimid- (CD) und/oder Uretonimin- (UI) Gruppen aufweisender Isocyanatmischungen mit niedriger Farbzahl, die nach diesem Verfahren erhältlichen Isocyanatmischungen

CD- und /oder UI-Gruppen aufweisende Isocyanatmischungen können in einfacher Weise mit den hoch wirksamen Katalysatoren aus der Phospholin-Reihe, insbesondere der Phospholinoxid-Reihe, nach den Verfahren gemäß US-A-2,853,473, US-A-6,120,699 US.A 4120884 und EP-A-515 933 hergestellt werden.

Die hohe katalytische Aktivität der Phospholinkatalysatoren, insbesondere der Phospholinoxidkatalysatoren, ist einerseits erwünscht, um die Carbodiimidisierungsreaktion unter schonenden Temperaturbedingungen anzustoßen, andererseits ist aber bis heute kein Verfahren bekannt, das eine wirksame Abstoppung der Phospholin-Katalyse bzw. der Phospholinoxid-Katalyse ohne Einschränkung gewährleistet. Die carbodiimidisierten Isocyanate neigen zur Nachreaktion, d.h. sie gasen infolge CO₂-Entwicklung aus. Dies führt dann besonders bei höheren Temperaturen zu einem Druckaufbau beispielsweise in den Lagerbehältern.

Es hat nicht an Versuchen gefehlt, eine wirksame Abstoppung der Phospholin-Katalyse zu finden. Derartige Stopper sind z.B. in den Patentschriften DE-A-25 37 685, EP-A-515 933, EP-A-609 698 und US-A-6,120,699 erwähnt und umfassen z.B. Säuren, Säurechloride, Chlorofomiate, silylierte Säuren und Halogenide der Hauptgrupperielemente. Ein Abstoppen des Katalysators mit Säuren, die z.B. auch als Säurechloride vorliegen können, ist nicht ausreichend wirksam.

Nach der Lehre der EP-A-515 933 werden mittels Phospholin-Katalyse hergestellte CD/UI-haltige Isocyanatmischungen mit mindestens der äquimolaren Menge, bevorzugt der 1-2fachen molaren Menge bezogen auf den eingesetzten Katalysator an z.B. Trimethylsilyltrifluormethansulfonat (TMST) abgestoppt. In der Praxis hat sich jedoch erwiesen, dass solchermaßen hergestellte CD/UI-enthaltende Isocyanate zur Herstellung von Prepolymeren, d.h. Umsetzungsprodukten von diesen CD/UI-enthaltenden Isocyanaten mit Polyolen, nur bedingt geeignet sind. Die entsprechend hergestellten Umsetzungsprodukte aus Polyolen und den CD/UI-modifizierten Isocyanaten neigen zum Ausgasen, was zu einem Druckaufbau in den Transportbehältern oder zum Schäumen bei der Handhabung derartiger Produkte führen kann.

Man kann dieses Problem dadurch umgehen, dass man die zum Abstoppen des Phospholin-Katalysators benutzte silylierte Säure analog der EP-A-515 933 in höheren molaren Äquivalenten (z.B. 5:1-10:1 bezogen auf den Katalysator) einsetzt. In der Praxis zeigt sich dann jedoch, dass die erhaltenen CD/UI modifizierten Isocyanate eine deutlich schlechtere Farbzahl aufweisen. Dies gilt dann auch für die hieraus hergestellten Prepolymere.

Dies gilt auch, wenn der Phospholinkatalysator mit Säuren vom Typ der Trifluornlethansulfonsäure entsprechend der US-A-6,120,699 abgestoppt wird. Auch daraus hergestellte Prepolymere weisen eine erheblich erhöhte Farbzahl auf.

Aufgabe der vorliegenden Erfindung war es daher, ein einfaches und wirtschaftliches Verfahren zur Herstellung flüssiger, lagerstabiler und heller Carbodiimid- und/oder Uretonimingruppen aufweisender Isocyanatmischungen zur Verfügung zu stellen, welches die angesprochenen Mängel nicht aufweist und zu flüssigen, lagerstabilen Isocyanatmischungen mit niedrigen Farbzahlen führt.

Die Erfindung betrifft ein Verfahren zur Herstellung Carbodiimid- und/oder Uretonimingruppen aufweisender organischer Isocyanate, bei dem ein oder mehrere organische Isocyanate mit einer Hazen-Farbzahl von ≤ 100 APHA, bevorzugt ≤ 50 APHA, mit Katalysatoren vom Phospholin-Typ teilweise carbodiimidisiert werden, und anschließend die Carbodiimidisierungsreaktion abgestoppt wird, dadurch gekennzeichnet, dass der Stopper in wenigstens zwei Portionen zugegeben wird, wobei die Temperatur des Reaktionsgemisches während der Zugabe der ersten Portion höher ist als die Temperatur des Reaktionsgemisches während der Zugabe der zweiten Portion. Dadurch wird ein helles Produkt erhalten.

Die Messung der Hazen-Farbzahl kann dabei gemäß DIN/EN/ISO 6271-2 (Entwurf September 2002) in Substanz gegen Wasser als Referenz bei einer Schichtdicke von 5 cm erfolgen. Als Messgerät kann z. B. ein Photometer Dr. Lange LICO 300 eingesetzt werden.

Selbstverständlich können auch organische Isocyanate mit einer höheren Farbzahl als Einsatzstoffe verwendet werden. In diesem Falle können allerdings die Vorteile hinsichtlich der günstigen Farbwerte nicht in vollem Umfang genutzt werden.

Nach dem erfindungsgemäßen Verfahren sind die Carbodiimid- und/oder Uretonimingruppen aufweisenden organischen Isocyanate erhältlich. Diese Carbodiimid- und/oder Uretonimingruppen aufweisenden organischen Isocyanate sind bei Raumtemperatur und in Abhängigkeit vom CD / UI-Gehalt und/oder vom eingesetzten Isocyanat bis hin zu tiefen Temperaturen (z.B. 0°C) flüssig.

Die nach erfindungsgemäßen Verfahren erhältlichen Carbodiimid- und/oder Uretonimingruppen aufweisenden organischen Isocyanate werden bevorzugt zur Herstellung von Abmischungen mit weiteren Isocyanaten bzw. zur Herstellung von Isocyanatgruppen enthaltenden Prepolymeren mit verbesserter Farbzahl verwendet.

Die nach erfindungsgemäßen Verfahren erhältlichen Carbodiimid- und/oder Uretonimingruppen aufweisenden organischen Isocyanate und die daraus hergestellten Isocyanat-Abmischungen und/oder Prepolymere mit verbesserter Farbzahl werden bevorzugt zur Herstellung von Polyurethankunststoffen verwendet.

Als Ausgangsmaterialien für das erfindungsgemäße Verfahren können beliebige organische Isocyanate mit einer Hazen-Farbzahl von ≤100 APHA, bevorzugt ≤ 50 APHA, eingesetzt werden. Vorzugsweise wird das erfindungsgemäße Verfahren jedoch zur Carbodiimidisierung von organischen Diisocyanaten verwendet, die in der Polyurethan-Chemie eingesetzt werden können.

Selbstverständlich können auch organische Isocyanate mit einer höheren Farbzahl als Einsatzstoffe verwendet werden. In diesem Falle können allerdings die Vorteile hinsichtlich der günstigen Farbwerte nicht in vollem Umfang genutzt werden.

Geeignete Isocyanate sind z.B. aromatische, araliphatische, aliphatische und/oder cycloaliphatische Diisocyante und/oder Polyisocyanate.

Als Vertreter der aliphatischen und/oder cycloaliphatischen Diisocyante sind beispielhaft zu nennen Isophorondiisocyanat, Hexamethylendiisocyanat und Dicyclohexylmethandiisocyanat (jeweils die reinen Isomeren sowie beliebige Isomerengemische).

Als Vertreter der araliphatischen Diisocyante sind beispielhaft zu nennen die verschiedenen Isomeren der Xylidendiisocyante.

Geeignet sind insbesondere aromatische Di- und Polyisocyanate wie Toluylendiisocyanat und Di-und Polyisocyanate der Diphenylmethanreihe.

Geeignet sind insbesondere:
- Aromatische Diisocyanate wie 2,4- und/oder 2,6-Diisocyanatotoluol (TDI), 2,2'-, 2,4'-und/oder 4,4'-Diisocyanatodiphenylmethan (MDI) bzw. beliebige Gemische derartiger aromatischer Diisocyanate,
- Di- und Polyisocyanatgemische der Diphenylmethanreihe mit einem Gehalt an monomeren Diisocyanatodiphenylmethan-Isomeren von 80 bis 100 Gew.-% und einem Gehalt an höher als difunktionellen Polyisocyanaten der Diphenylmethanreihe von 0 bis 20 Gew.-%, wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen.

Als Ausgangsmaterialien bevorzugte organische Isocyanate sind insbesondere aromatische Diisocyanate wie 2,4- und/oder 2,6-Diisocyanatotoluol (TDI), 2,2'-, 2,4'- und/oder 4,4' flüsocyanatodiphenylmethan (MDI) bzw. beliebige Gemische derartiger aromatischer Diisocyanate. Besonders bevorzugt sind 2,2'-, 2,4'- und/oder 4,4'-Diisocyanatodiphenylmethan (MDI) bzw. beliebige Gemische derartiger aromatischer Diisocyanate, wobei die Summe an 2,2'-, 2,4'-und/oder 4,4'-Diisocyanatodiphenylmethan im Ausgangsmaterial (organisches Isocyanat) mindestens 85 Gew.-% beträgt, und wobei sich die Düsocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen. Ganz besonders bevorzugt sind 2,2'-, 2,4'- und/oder 4,4'-Diisocyanatodiphenylmethan (MDI) bzw. beliebige Gemische aromatischer Diisocyanate, wobei die Summe an 2,2'-, 2,4'- und/oder 4,4'-Diisocyanatodiphenylmethan im Ausgangsmaterial (organisches Isocyanat) mindestens 90 Gew.-% beträgt, und wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen. Insbesondere ganz besonders bevorzugt sind 2,2'-, 2,4'- und/oder 4,4'-Diisocyanatodiphenylmethan (MDI) bzw. beliebige Gemische aromatischer Diisocyanate, wobei die Summe an 2,2'-, 2,4'- und/oder 4,4'-Diisocyanatodiphenylmethan im Ausgangsmaterial (organisches Isocyanat) mindestens 99 Gew.-% beträgt, und wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen.

Das erfindungsgemäße Verfahren wird in Gegenwart von Katalysatoren vom Phospholin-Typ durchgeführt. Die Katalysatoren vom Phospholin-Typ sind beispielsweise aus EP-A-515 933 und US-A-6,120,699 bekannt. Typische Beispiele dieser Katalysatoren sind beispielsweise die aus dem Stand der Technik bekannten Gemische der Phospholinoxide der Formel:

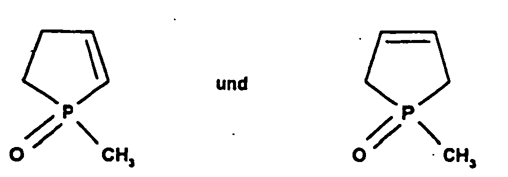

Die Menge des eingesetzten Katalysators hängt von der Qualität und/oder der Reaktivität der Ausgangsisocyanate ab. Die jeweils notwendige Katalysatormenge lässt sich daher am einfachsten in einem Vorversuch bestimmen.

Die Carbodiimidisierungsreaktion wird üblicherweise im Temperaturbereich zwischen 50 bis 150°C, vorzugsweise von 60 bis 100°C, durchgeführt. Jedoch sind auch deutlich höhere Reaktionstemperaturen möglich (bis zu ca. 280°C). Die optimale Reaktionstemperatur richtet sich nach der Art der Ausgangsisocyanate und / oder des eingesetzten Katalysators und kann in einem einfachen Vorversuch ermittelt werden.

Die Carbodiimidisierungsreaktion wird im allgemeinen bei Erreichen eines Carbodiimidisierungsgrades (Carbodiimidisierungsgrad ist der Prozentsatz der carbodiimidisierten Isocyanatgruppen bezogen auf die Gesamtmenge der in Ausgangsisocyanat vorliegenden Isocyanatgruppen) von 3 bis 50 %, vorzugsweise 5 bis 30 %, abgebrochen.

Der Carbodiimidisierungsgrad kann während der Durchführung des erfindungsgemäßen Verfahrens durch Bestimmung des NCO-Wertes z.B. mittels dem Fachmann an sich bekannter Titration oder mittels online-Verfahren bestimmt werden. Ein geeignetes online-Verfahren ist z.B. die Nahinfrarot- oder die Mittelinfrarot-Analytik.

Der Carbodiimidisierungsgrad kann während der Durchführung des erfindungsgemäßen Verfahrens ebenfalls z.B. an der Menge des im Reaktorgemisch entweichenden Kohlendioxids erkannt werden. Diese volumetrisch bestimmbare Kohlendioxidmenge gibt somit zu jedem Zeitpunkt Auskunft über den erreichten Carbodiimidisierungsgrad.

Darüber hinaus können grundsätzlich auch andere geeignete, dem Fachmann bekannte offline-oder online-Methoden der Prozessverfolgung eingesetzt werden.

Zum Beenden der Carbodiimidisierungsreaktion wird bevorzugt mindestens die äquimolare Menge, besonders bevorzugt der 1 - 20 fache molare Überschuss, ganz besonders bevorzugt der 1 - 10 fache molare Überschuss, bezogen auf den Katalysator, eines Abstoppers, bevorzugt Trimethylsilyltrifluormethansulfonat (TMST) oder eines Alkylierungsmittels oder eines Gemisches der genannten Abstopper eingesetzt. Bevorzugt wird dabei ein Alkylierungsmittel oder Trimethylsilyltrifluormethansulfonat (TMST) als einziger Abstopper eingesetzt.

Bevorzugte Alkylierungsmittel sind Ester der Trifluormethansulfonsäure, Ester anorganischer Säuren (bevorzugt starker anorganischer Säuren) oder Trialkyloxoniumverbindungen.

Die Carbodiimidisierungsreaktion wird abgestoppt, indem der Stopper in wenigstens zwei Portionen zugegeben wird, wobei die Temperatur des Reaktionsgemisches während der Zugabe der ersten Portion höher ist als die Temperatur des Reaktionsgemisches während der Zugabe der zweiten Portion. Bevorzugt ist bei der Zugabe des Stoppers in mehr als zwei Portionen die Temperatur des Reaktionsgemisches während der Zugabe der ersten Portion höher als die Temperatur des Reaktionsgemisches während der Zugabe aller weiteren Portionen. Bevorzugt liegt die Temperatur des Reaktionsgemisches während der Zugabe der ersten Portion des Stoppers mindestens 20°C, besonders bevorzugt mindestens 30°C, oberhalb der Temperatur des Reaktionsgemisches während der Zugabe der zweiten Portion an Stoppers und bevorzugt auch aller weiteren Portionen an Stopper.

Bevorzugt erfolgt die Abstoppung durch Zugabe des Stoppers in zwei bis fünf Portionen, besonders bevorzugt zwei, drei oder vier Portionen. Die erst Portion an Stopper ist bevorzugt so bemessen, dass mindestens die der molaren Katalysatormenge entsprechende molare Menge des Stoppers zugegeben wird. In der zweiten und den ggf. weiteren Portionen der Stopperzugabe wird dann zusätzlicher Stopper zugegeben.

Bevorzugt liegt zwischen der Zugabe der ersten Portion an Stopper und der Zugabe der zweiten Portion an Stopper ein zeitlicher Abstand von mindestens 1 min, besonders bevorzugt mindestens 5 min, ganz besonders bevorzugt mindestens 10 min.

Das Reaktionsprodukt der Carbodiimidisierung kann Farbstabilisatoren enthalten, wie sie üblicherweise Isocyanaten zugesetzt werden. Dabei ist der Zeitpunkt des Zusatzes nicht kritisch. Die Farbstabilisatoren können entweder dem als Ausgangmaterial verwendeten Isocyanat vor der Carbodiimidisierung zugesetzt werden, oder dem Reaktionsprodukt nach vollendeter Umsetzung. Es ist ebenfalls möglich, Farbstabilisatoren sowohl dem Ausgangsmaterial als auch dem Reaktionsprodukt zuzugeben. Derartige Stabilisatoren sind generell dem Fachmann bekannt und umfassen z.B. Substanzen aus der Gruppe der sterisch gehinderten Phenole, der Phosphorigsäureester oder der sterisch gehinderten Amine. Die Farbstabilisatoren können jeweils für sich allein oder im Gemisch mit anderen Vertretern der gleichen oder verschiedener Substanzgruppen eingesetzt werden. Die Mengen der eingesetzten Farbstabilisatoren bewegen sich in der dem Fachmann bekannten Größenordnung, üblicherweise im Bereich von 100 ppm bis 10000 ppm für die Einzelsubstanz bzw. das Gemisch, bezogen auf das als Ausgangsmaterial verwendete Isocyanat bzw das Reaktionsprodukt der Carbodiimidisierung.

Isocyanatgruppen enthaltende Prepolymere werden durch Umsetzung der nach dem erfindungsgemäßen Verfahren hergestellten Carbodiimid- und/oder Uretonimingruppen aufweisenden organischen Isocyanate mit in der Polyurethanchemie üblichen Polyolen erhalten. Geeignete Polyole sind sowohl einfache mehrwertige Alkohole des Molekulargewichtsbereiches 62 bis 599 g/mol, vorzugsweise 62 bis 300 g/mol, wie z.B. Ethylenglykol, Trimethylolpropan, Propandiol-1,2, Butandiol-1,2 oder Butandiol-2,3, Hexandiol, Octandiol, Dodecandiol und/oder Octadecandiol, insbesondere jedoch höhermolekulare Polyetherpolyole und/oder Polyesterpolyole der aus der Polyurethanchemie an sich bekannten Art mit Molekulargewichten von 600 bis 8000 g/mol, vorzugsweise 800 bis 4000 g/mol, die mindestens zwei, in der Regel 2 bis 8, vorzugsweise 2 bis 4 primäre und/oder sekundäre Hydroxylgruppen aufweisen. Beispiele derartiger Polyole sind in der US-PS 4 218543, Kolonne 7, Zeile 29 bis Kolonne 9, Zeile 32 beschrieben.

Die Vorteile des erfindungsgemäßen Verfahrens sind augenscheinlich: Durch die Zugabe des Stoppers in zwei oder mehr Portionen auf unterschiedlichem Temperaturniveau weisen sowohl die Carbodiimid- und/oder Uretonimingruppen-haltigen Isocyanate als auch die daraus hergestellten Prepolymere eine helle Farbe auf.

Diese Carbodiimid- und/oder Uretonimingruppen aufweisenden organischen Isocyanate und die daraus durch Umsetzung mit Polyolen hergestellten Prepolymere stellen wertvolle Ausgangsmaterialien zur Herstellung von Polyurethankunststoffen durch Umsetzung mit Polyolen (z.B. mit Polyetherpolyolen oder Polyesterpolyolen) nach dem Isocyanat-Polyadditionsverfahren dar.

### Beispiele:

### Ausgangsprodukte:

- Desmodur 44M^{®}, Bayer AG (4,4'- Diphenylmethandiisocyanat, NCO-Gehalt: 33,6 Gew.-%)
- Katalysator vom Phospholinoxid-Typ: technisches Gemisch aus 1-Methyl-1-oxo-1-phosphacyclopent-2-en und 1-Methyl-1-oxo-1-phosphacyclopent-3-en, 1 Gew.-%ig in Toluol
- Stopper: Trifluormethansulfonsäureethylester (TFMSEE) bzw. Trimethylsilyltrifluormethansulfonat (TMST)

Allgemeine Vorschrift zur Herstellung des Carbodiimid- und/oder Uretonimingruppen aufweisenden organischen Isocyanates:

10 kg technisches 4,4'-MDI (Desmodur 44M^{®}, der Bayer MaterialScience AG) mit einer Hazen Farbzahl von < 15 APHA, das 750 ppm 3,5-Di-*tert*-butyl-4-hydroxytoluol enthält, werden unter N₂/Rühren auf ca. 90°C erhitzt. Anschließend werden 2,5 ppm Katalysator in Form einer 1 %igen Lösung in Toluol zugegeben. Das Reaktionsgemisch wird unter N₂/Rühren bis zum Erreichen des erwünschten NCO-Gehaltes auf ca. 95°C erhitzt. Danach wird die Carbodiimidisierung durch Zugabe von 10 ppm des jeweiligen Stoppers (Trifluormethansulfonsäureethylester (TFMSEE) bzw. Trimethylsilyltrifluormethansulfonat (TMST); siehe Tabelle) abgestoppt und 1 Stunde nachgerührt. Eine zweite Stoppermenge (siehe Tabelle) wird bei Raumtemperatur zugegeben.

Die Ergebnisse sind in der nachstehenden Tabelle zusammengefasst.

Die Messung der Hazen-Farbzahl erfolgt gemäß DIN/EN/ISO 6271-2 (Entwurf September 2002) in Substanz gegen Wasser als Referenz bei einer Schichtdicke von 5 cm. Als Messgerät kann z. B. ein Photometer Dr. Lange LICO 300 eingesetzt werden.

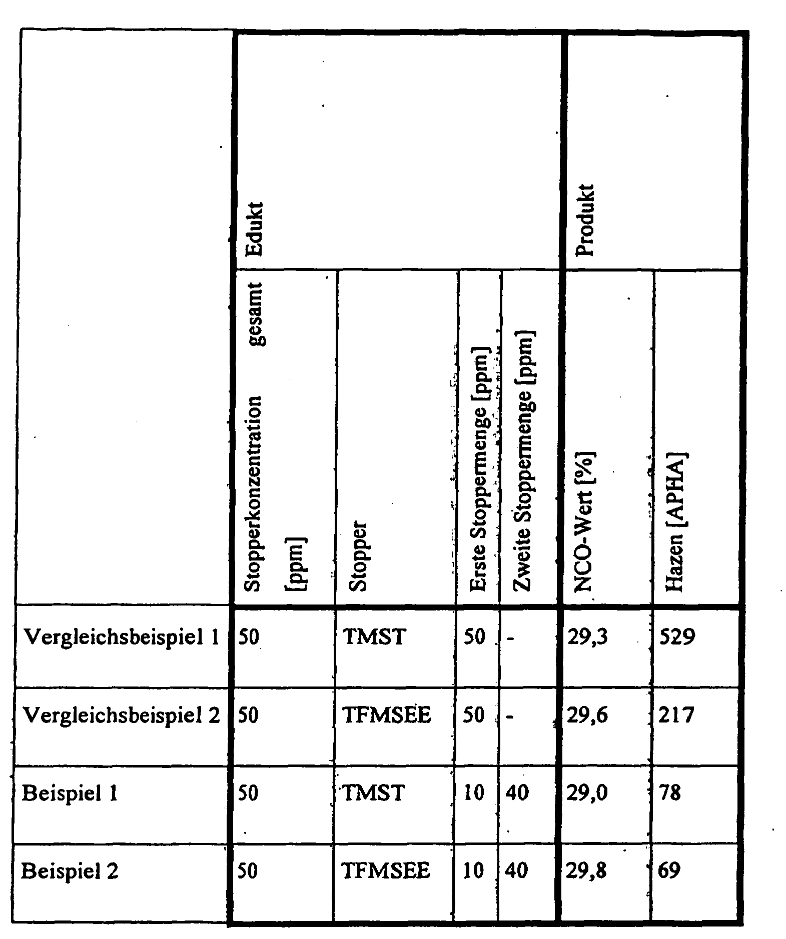

Die erfindungsgemäßen Beispiele verdeutlichen den gegenüber den Vergleichsbeispielen vorteilhaften Effekt der mehrstufigen Stopperzugabe hinsichtlich der Produktfarbe.

## Patentansprüche

1. Verfahren zur Herstellung Carbodiimid- und/oder Uretonimingruppen aufweisender organischer Isocyanate, bei dem ein oder mehrere organische Isocyanate mit einer Hazen-Farbzahl von ≤ 100 APHA mit Katalysatoren vom Phospholin-Typ teilweise carbodiimidisiert werden, und anschließend die Carbodiimidisierungsreaktion abgestoppt wird, **dadurch gekennzeichnet, dass** der Abstopper in wenigstens zwei Portionen zugegeben wird, wobei die Temperatur des Reaktionsgemisches während der Zugabe der ersten Portion höher ist als die Temperatur des Reaktionsgemisches während der Zugabe der zweiten Portion.

2. Verfahren nach Anspruch 1, **dadurch gekennzeichnet, dass** die Temperatur des Reaktionsgemisches während der Zugabe der ersten Portion des Abstoppers mindestens 20°C oberhalb der Temperatur des Reaktionsgemisches während der Zugabe der zweiten Portion an Abstopper liegt.

3. Verfahren nach Anspruch 1, **dadurch gekennzeichnet, dass** die Temperatur des Reaktiongemisches während der Zugabe der ersten Portion des Abstopper mindestens 30°C oberhalb der Temperatur des Reaktionsgemisches während der Zugabe der zweiten Portion an Abstopper liegt.

## Claims

1. Process for the preparation of organic isocyanates containing carbodiimide and/or uretonimine groups, in which one or more organic isocyanates with a Hazen colour number of ≤ 100 APHA are partially carbodiimidized using catalysts of the phospholine type and the carbodiimidization reaction is then terminated, **characterized in that** the terminating agent is added in at least two portions, the temperature of the reaction mixture during the addition of the first portion being higher than the temperature of the reaction mixture during the addition of the second portion.

2. Process according to claim 1, **characterized in that** the temperature of the reaction mixture during the addition of the first portion of the terminating agent is at least 20°C above the temperature of the reaction mixture during the addition of the second portion of the terminating agent.

3. Process according to claim 1, **characterized in that** the temperature of the reaction mixture during the addition of the first portion of the terminating agent is at least 30°C above the temperature of the reaction mixture during the addition of the second portion of the terminating agent.

## Revendications

1. Procédé de préparation d'isocyanates organiques comportant des groupes carbodiimido et/ou urétonimino, dans lequel un ou plusieurs isocyanates organiques ayant un indice de coloration Hazen égal ou inférieur à 100 APHA sont partiellement carbodiimidés avec des catalyseurs de type phospholine, la réaction de carbodiimidation étant ensuite stoppée, **caractérisé en ce que** le stoppeur est ajouté en deux portions au moins, la température du mélange réactionnel au cours de l'adjonction de la première portion étant supérieure à celle du mélange réactionnel au cours de l'adjonction de la seconde portion.

2. Procédé selon la revendication 1, **caractérisé en ce que** la température du mélange réactionnel au cours de l'adjonction de la première portion du stoppeur est au moins supérieure de 20 °C à celle du mélange réactionnel au cours de l'adjonction de la seconde portion du stoppeur.

3. Procédé selon la revendication 1, **caractérisé en ce que** la température du mélange réactionnel au cours de l'adjonction de la première portion du stoppeur est au moins supérieure de 30 °C à celle du mélange réactionnel au cours de l'adjonction de la seconde portion du stoppeur.
